(19) Europäisches Patentamt
European Patent Office
Office européen des brevets

(11) Veröffentlichungsnummer: **0 260 598 B1**

(12) **EUROPÄISCHE PATENTSCHRIFT**

(45) Veröffentlichungstag der Patentschrift: **16.10.91**

(51) Int. Cl.[5]: **C07D 311/72**, C07D 307/32, C07C 69/618, A61K 7/40

(21) Anmeldenummer: **87113235.3**

(22) Anmeldetag: **10.09.87**

# (54) Lichtschutzmittel mit einem Gehalt an ungesättigten Estern.

(30) Priorität: **15.09.86 CH 3696/86**
**16.07.87 CH 2714/87**

(43) Veröffentlichungstag der Anmeldung:
**23.03.88 Patentblatt 88/12**

(45) Bekanntmachung des Hinweises auf die Patenterteilung:
**16.10.91 Patentblatt 91/42**

(84) Benannte Vertragsstaaten:
**AT BE CH DE ES FR GB IT LI NL**

(56) Entgegenhaltungen:

**DERWENT JAPANESE PATENT REPORTS, Band 10, Nr. 90, (C-337)[2147], 8. April 1986; & JP-A-60 222 476 (ICHIMARU FUARUKOSU K.K.) 07-11-1985**

(73) Patentinhaber: **F. HOFFMANN-LA ROCHE AG**
**Postfach 3255**
**CH-4002 Basel(CH)**

(72) Erfinder: **Erlemann, Gustav, Dr.**
**Chrischonastrasse 60**
**CH-4058 Basel(CH)**
Erfinder: **Fizet, Christian, Dr.**
**18 rue de Bruebach**
**F-68440 Zimmersheim(FR)**
Erfinder: **Pauling, Horst, Dr.**
**Ruchholzstrasse 39**
**CH-4103 Bottmingen(CH)**

(74) Vertreter: **Urech, Peter, Dr. et al**
**Grenzacherstrasse 124 Postfach 3255**
**CH-4002 Basel(CH)**

**EP 0 260 598 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Verbindungen, nämlich p-Methoxy-zimtsäureester der allgemeinen Formel

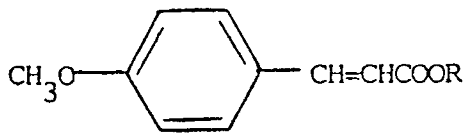

I

worin R den Alkoholrest von d,l-α-Tocopherol, Pantolacton oder Panthenol darstellt, wobei Panthenol ein-, zwei- oder dreifach verestert sein kann,
und ein Verfahren zu deren Herstellung.

Die Begriffe "Pantolacton" und "Panthenol" sollen die <R> und die <R,S>-Formen umfassen.

Die Ester der Formel I absorbieren die erythem-erzeugenden Ultraviolettstrahlen der Sonne (zwischen 270 uni 330 mμ). d.h. sie halten diese Strahlen von den lebenden Epidermis-Zellen fern. Die Ester der Formel I können infolgedessen als Lichtschutzmittel Verwendung finden. Die Erfindung betrifft demgemäss auch ein Verfahren zur Herstellung eines Lichtschutzmittels, das dadurch gekennzeichnet ist, dass man eine Verbindung der Formel I in eine in der Kosmetik übliche Grundlage einarbeitet. Die Erfindung betrifft ferner ein Lichtschutzmittel, das durch einen Gehalt an einer Verbindung der Formel I gekennzeichnet ist und schliesslich die Verwendung der Verbindungen I als Lichtschutzmittel.

Die Verbindungen I können erfindungsgemäss dadurch erhalten werden, dass man ein reaktives Derivat der p-Methoxyzimtsäure, insbesondere das Chlorid, in an sich bekannter Weise verestert.

Zweckmässig werden die Ausgangsmaterialien in reiner Form eingesetzt.

Die Veresterung wird in an sich bekannter Weise unter Verwendung eines reaktiven Derivats, z.B. des Anhydrids, oder - vorzugsweise - eines Halogenids, insbesondere des Chlorids, durchgeführt. Man arbeitet dabei zweckmässigerweise in Anwesenheit von tertiären Aminen, wie z.B. Pyridin, Triäthylamin oder N,N-Dimethylanilin. Zugabe eines Lösungsmittels, z.B. eines (chlorierten) Kohlenwasserstoffs, oder eines niederen Esters ist zweckmässig.

Als kosmetische Grundlage kann jede übliche Zubereitung dienen, die den kosmetischen Anforderungen entspricht, z.B. Oele, Crèmes, Lotions, Emulsionen, Salben, Gele, Lösungen. Sprays, Sticks und dgl. Die Lichtschutzwirkung ist natürlich von der verwendeten Grundlage abhängig. Die Intensität der Lichtschutzwirkung hängt weiter bei gleicher Grundlage von der Wirkstoffkonzentration ab. Geeignete Konzentrationen sind z.B. zwischen 1-6%, vorzugsweise zwischen 3-5% im kosmetischen Präparat.

Die erfindungsgemässen Substanzen können auch mit anderen üblichen Lichtschutzmitteln kombiniert werden, wobei sogar eine Potenzierung der Lichtschutzwirkung, d.h. ein synergistischer Effekt auftreten kann.

Erwähnenswert ist des weitern die ausgesprochene Hautfreundlichkeit der neuen Ester.

Eine zusätzliche wertvolle Eigenschaft der Verbindungen I besteht darin, dass sie die Epithelisierung von oberflächlichen Wunden beschleunigen und somit, ganz allgemein, die Wundheilung fördern. Diese Tatsache ist deshalb von Bedeutung, weil oft vom Verbraucher nicht genügend des Sonnenschutzmittels aufgetragen wird, oder ein Sonnenschutzpräparat mit ungenügendem Sonnenschutzfaktor benützt wird. Es resultiert in der Folge ein Sonnenbrand. Die Verbindungen I beschleunigen in solchen Fällen das Abklingen des Sonnenbrandes ganz ausgesprochen.

Die Beeinflussung der Epithelisierung und Wundheilung wurde mit der folgenden Methodik nachgewiesen:

Es wurde ein Wundheilungsmodell entwickelt, das die Beurteilung der Epithelisierung und Heilung oberflächlicher Wunden ermöglicht. Die gute Reproduzierbarkeit der Methode geht aus wiederholten Vergleichen zwischen Placebo und Präparaten mit D-Panthenol als Wirkstoff hervor.

### Material

Für die Untersuchung wurde ein Gel folgender Zusammensetzung mit und ohne den Panthenoltriester der p-Methoxyzimtsäure hergestellt:

## Placebo

| | |
|---|---|
| Pluronic L 62 (Emulgator) | 10.00 g |
| Lecithin für Mischmicellen (zwecks Erzielen homogener Gele) | 0.50 g |
| Aerosil 200 ($SiO_2$: Konsistenzgeber) | 8.00 g |
| Triglycerid mittelkettig (Gelbildner) | 81.50 g |
| | 100.00 g |

## Placebo mit Panthenoltriester der p-Methoxyzimtsäure

| | |
|---|---|
| Panthenoltriester der p-Methoxyzimtsäure | 1.00 g |
| Pluronic L 62 | 10.00 g |
| Lecithin für Mischmicellen | 0.50 g |
| Aerosil 200 | 8.00 g |
| Triglycerid mittelkettig | 80.50 g |
| | 100.00 g |

### Versuchstiere und Haltung der Tiere

Die Wundheilungversuche wurden mit männlichen Füllinsdorf-Albino-Ratten der SPF-Zucht des Instituts für Biologisch-Medizinische Forschung AG, 4414 Füllinsdorf durchgeführt. Ihr Durchschnittsgewicht bei der Ankunft im Labor betrug zirka 200 g. Die Tiere wurden unter einwandfreien hygienischen Bedingungen und vor Zugluft geschützt in Einzelkäfigen gehalten.

### Futterrationen

Zwecks Ausschaltung des Einflusses von Vitaminzusätzen auf die Wundheilung wurde ein halbsynthetisches Futter zubereitet, das den Anforderungen an Standard-Rattenfutter entspricht. Die Futterrationen enthielten 12,6 MJ verwertbare Energie pro kg und 17% verdaubares Roheiweiss. Bei Prüfungen mit den topischen Präparaten wurde pelletiertes Rattenfutter NAFAG 854 (Nafag Nähr- und Futtermittel AG. 9202 Gossau) verabreicht.

### Blistertechnik

Um den Heilungsverlauf bei topischer Anwendung der Präparate Prüfen zu können, wurden Saugnäpfe zur Erzeugung der intraepidermalen Wunden eingesetzt. Bedingt durch die Anordnung der Saugnäpfe wurde auf beiden Seiten der linea alba je eine Wunde im Abstand von 20 mm und einer Fläche von 12.6 $mm^2$ gesetzt. Eine gemeinsame Vakuumleitung sorgte für identische Bedingungen an den Saugnäpfen. Der Unterdruck wurde durch eine Handpumpe (Dermovac, Firma Instrumentarium, Helsinki, Finnland) erzeugt, durch Manometer und Nadelventil kontrolliert bzw. reguliert. Nach einer Stunde Unterdruck von 100 mg Hg-Säule entstand eine Blase, deren Inhalt mit Hilfe einer Pasteur-Pipette für analytische Zwecke gewonnen wurde. Für die Anaesthesie wurde Thiogenal R für Tiere verwendet, ein exzitationsloses Thiobarbiturat der Firma Merck GmbH, Darmstadt.

Dampfdruckmessung

Die seröse Flüssigkeit auf die Wundfläche wurde mit Hilfe schnell ansprechender Sensoren gemessen. Zur simultanen Messung von intakter Hautstelle (oberhalb Xiphoid) und den beiden Wundflächen sind drei Sensoren in einem Messkopf untergebracht. Um für alle drei Messstellen identische Ausgangsbedingungen zu erhalten, wurden die Kammern nach der Positionierung mit einem über Molekularsieb getrockneten Luftstrom auf 15% Restfeuchtigkeit eingestellt. Die Dampfdrucksättigungskurven der drei Messstellen wurden unabhängig voneinander auf einem 3-Kanal-Linienschreiber registriert. Die Messdauer betrug jeweils exakt 2 1/2 Minuten bei einem Papiervorschub von 4 cm•Min$^{-1}$. Aus den Diagramm-Aufzeichnungen wurde die relative Feuchtigkeitsabgabe ermittelt, indem jeweils die Distanz der Kurvenabschnitte für die beiden Wundflächen bestimmt und durch den Messwert an der intakten Hautstelle korrigiert wurde.

Das Auftragen von Präparat (linke Bauchseite) und Placebo (rechts) erfolgte täglich im Anschluss an den Messvorgang. Mit einem kleinen Spatel wurden 0,75 mg der jeweiligen Formulierung pro Wundfläche fein verteilt. Für den schnellen Messvorgang genügte die kurz wirksame Inhalationsnarkose mit Halothan R von der Firma Höchst, Frankfurt.

Statistische Auswertung der Messergebnisse

Um den Heilungsverlauf besser verfolgen zu können, wurden die bei der Wundsetzung erzielten ersten Messwerte auf 100% gesetzt. Die zu jedem Zeitpunkt erhaltenen Ergebnisse einer Gruppe wurden für die Bildung von Mittelwert und Standardfehler herangezogen. Die lineare Regressionsrechnung wurde angewendet, um die Zeit bis zum völligen Wundverschluss angeben zu können. Die Heildauer in Stunden wird durch den x-Achsenabschnitt ausgedrückt, der beim Schnitt der Regressionsgeraden mit der Abszisse entsteht. Mit der Berechnung des Quotienten aus den x-Achsenabschnitten wurde die Wirksamkeit der Versuchscrème ermittelt, die der Beschleunigung der Heilverlaufes entspricht.

Ergebnisse

Die Wirkung des Zusatzes von Panthenoltriester der p-Methoxyzimtsäure auf die Epithelisierung oberflächlicher Wunden konnte an Hand der Versuche einwandfrei nachgewiesen werden. Im Vergleich zum Placebo zeigte das Gel mit 1% Zusatz des Panthenoltriesters der p-Methoxyzimtsäure eine signifikante Beschleunigung der Epithelisierung mit dem Faktor 1.3.

Der Panthenol-di- und der -monoester zeigten ein analoges Verhalten.

In den folgenden Beispielen sind die Temperaturen in Celsiusgraden angegeben.

Beispiel 1

In einem 350 ml 4-Halssulfierkolben, versehen mit Thermometer, Rückflusskühler, Magnetrührer sowie 20 ml-Tropftrichter werden unter Argonbegasung 16,3 g <R>-Pantolacton (0.125 Mol) und 28 g 4-Methoxyzimtsäurechlorid (ca. 0,14 Mol) in 170 ml $CH_2Cl_2$ vorgelegt. Eine Lösung von 11 (0,139 Mol) g Pyridin in 30 ml $CH_2Cl_2$ wird nun innert 30 Minuten zugetropft, wobei die Temperatur langsam ansteigt. Das Reaktionsgemisch wird schlussendlich während 3 Stunden auf Rückflusstemperatur erhitzt. Das Reaktionsgemisch wird mit 150 ml Wasser, 2x mit 40 ml 5%iger HCl, 2x mit 40 ml 5%iger $NaHCO_3$ und lx mit 100 ml Wasser gewaschen. Die organische Phase wird abgetrennt und über $Na_2SO_4$ getrocknet. Das Lösungsmittel wird am Rotationsverdampfer entfernt. Das so erhaltene Rohprodukt wird aus 100 ml Essigester/Hexan (60:100) umkristallisiert. Man erhält 33,6 g weisse Kristalle des <R>-Pantolactonesters der p-Methoxyzimtsäure (Ausbeute 92,6%).

Beispiel 2

In einem 250 ml 4-Halssulfierkolben, versehen mit Thermometer und Magnetrührer werden unter Argonbegasung 34,8 g des obigen Esters (0.12 Mol) und 13,5 g 3-Aminopropanol (0,18 Mol) in 100 ml MeOH suspendiert bzw. gelöst. Nach ca. 30 Minuten wird das Reaktionsgemisch klar. Es wird während 3 Stunden bei Rückflusstemperatur gerührt. Das Methanol wird am Rotationsverdampfer abgedampft und der Rückstand in 350 ml $CH_2Cl_2$ gelöst. Diese Lösung wird mit 2x 60 ml Wasser gewaschen. Die organische Phase wird abgetrennt, über $Na_2SO_4$ getrocknet und am Rotationsverdampfer eingeengt. Der gelbe, viskose Rückstand (41.5 g) wird an 1 kg Kieselgel (0.040-0,063 mm) unter 0,5 bar Druck chromatographiert (Essigester/Acetonitril, 5:5). Man erhält 33,1 g leicht gelb gefärbten <R>-Panthenolmonoester der p-Methoxyzimtsäure (Ausbeute 75,4%).

Beispiel 3

In einem 500 ml 4-Halssulfierkolben, versehen mit Thermometer, 100 ml Tropftrichter, Magnetrührer und Rückflusskühler werden unter Argonbegasung 11,3 g <R>-Panthenol (0,055 Mol) in 100 ml Tetrahydrofuran vorgelegt. Dann wird innert ca. 15 Minuten eine Lösung von 27,8 g 4-Methoxyzimtsäurechlorid (0,14 Mol) in 100 ml THF zugetropft. Nun wird eine Lösung von 11.1 g Pyridin (0,14 Mol, innert ca. 30 Minuten zugetropft und noch 3 Stunden gerührt. Der Niederschlag wird abgenutscht und das Filtrat am Rotationsverdampfer eingeengt. Der Rückstand wird in 300 ml $CH_2Cl_2$ gelöst. Diese Lösung wird mit 2× 60 ml 5%iger HCl, dann mit 2× 60 ml 5%iger $NaHCO_3$ gewaschen. Nach Trocknung ($Na_2SO_4$) wird die organische Phase am Rotationsverdampfer eingeengt. Der gelbe, viskose Rückstand (33.8 g) wird an 1 kg Kieselgel (0,04-0,063 mm) unter 0,5 bar Druck chromatographiert (Toluol/Essigester, 8:2). Man erhält 15,9 g gelblichen <R>-Panthenoldiester der p-Methoxyzimtsäure (Ausbeute 55%).

Beispiel 4

In einem 750 ml 4-Halssulfierkolben. versehen mit Thermometer, 100 ml Tropftrichter, Magnetrührer und Rückflusskühler werden unter Argonbegasung 17,4 g <R>-Panthenol (0.085 Mol) in 300 ml Methylacetat vorgelegt. Dann wird innert ca. 20 Minuten eine Lösung von 75,5 g 4-Methoxyzimtsäurechlorid (0,38 Mol) in 150 ml Methylacetat zugetropft. Eine Lösung von 33,6 g Pyridin (0,425 Mol) wird innert ca. 40 Minuten zugetropft. Das Reaktionsgemisch wird schlussendlich 4 Stunden auf Rückflusstemperatur erhitzt. Nach Kühlung wird der Niederschlag abgenutscht. Das Filtrat wird mit 2× 150 ml 5%iger HCl, dann mit 2× 100 ml Wasser gewaschen. Nach Trocknung über $Na_2SO_4$ wird die organische Phase am Rotationsverdampfer eingeengt. Der gelbe, viskose Rückstand (74,2 g) wird an 1 kg Kieselgel (0,040-0,063 mm) unter 0.5 bar Druck chromatographiert (Toluol/Essigester, 7:1). Man erhält 50,1 g gelben <R>-Panthenoltriester der p-Methoxyzimtsäure (Ausbeute 86%).

Beispiel 5

In einem 100 ml 4-Halssulfierkolben, versehen mit Thermometer, 20 ml Tropftrichter, Magnetrührer und Rückflusskühler werden unter Argonbegasung 4,3 g dl-α-Tocopherol (0.010 Mol) und 1.34 g Pyridin (0,017 Mol) in 20 ml $CH_2Cl_2$ vorgelegt. Eine Lösung von 2,95 g 4-Methoxyzimtsäurechlorid (0.015 Mol) in 15 ml $CH_2Cl_2$ wird innert 10 Minuten zugetropft. Das Reaktionsgemisch wird während 3 Stunden auf Rückflusstemperatur erhitzt. Die Lösung wird nacheinander mit 10 ml Wasser, 10 ml 5%iger HCl und 10 ml 5%iger $NaHCO_3$ gewaschen. Nach Trocknung ($Na_2SO_4$) wird das Lösungsmittel am Rotationsverdampfer eingeengt. Das Rohprodukt (6.3 g) wird an 150 g Kieselgel (0,040-0,063 mm) unter 0,5 bar Druck chromatographiert (Toluol). Man erhält 5,2 g gelblichen d,l-α-Tocopherolester der p-Methoxyzimtsäure (Ausbeute 88%).

Beispiel 6

## A. Sonnenschutzlotion

| | | Gewichtsteile |
|---|---|---|
| A. | Panthenoldiester der p-Methoxyzimtsäure | 4,00 |
| | Isopropylmyristat | 3,00 |
| | Stearinsäure | 3,00 |
| | Mandelöl | 2,50 |
| | Hexyl-laurat | 8,00 |
| | Dimethicone (Siliconöl) | 1,00 |
| | Vit. E-acetat | 1,00 |
| B. | Diäthanolamin-cetyl-phosphat | 4,00 |
| C. | Allantoin | 0,30 |
| | Wasser | 73,20 |
| D. | Parfüm, Konservierungsmittel | q.s. |

A wird unter Rühren auf 85° C erhitzt. Anschliessend wird B in A gelöst. Dann wird C auf 70° C erwärmt und unter Rühren A+B zugefügt. Unter Rühren wird langsam abgekühlt und D bei 40° C zugegeben. Es wird bis zum Erreichen der Zimmertemperatur weitergerührt.

## B. Wasserresistente Schutzcrème (O/W Emulsion)

| | | Gewichtsteile |
|---|---|---|
| A. | Panthenolmonoester der p-Methoxyzimtsäure | 2 |
| | ARLACEL 481 (Sorbitansequioleat, Glycerinoleat und Bienenwachs) | 9 |
| | Lanolin | 1 |
| | Paraffinöl | 8 |
| | Vit. E-acetat | 2 |

| | | |
|---|---|---|
| B. | Aluminiumstearat | 0,1 |
| | Isopropylmyristat | 10,00 |
| C. | Magnesiumstearat | 0,3 |
| | Propylenglykol | 3 |
| | Wasser | 64,60 |
| D. | Parfüm und Konservierungsmittel | q.s. |

A wird unter Rühren bei 85° C gelöst. B wird bei 80° C gelöst und in A eingearbeitet. C wird auf 70° C erwärmt und unter Rühren dem Gemisch A+B beigefügt. Es wird langsam abgekühlt und D bei 40° C unter Rühren eingearbeitet. Es wird bis zur Erreichung der Zimmertemperatur weitergerührt.

### C. Sonnenschutz-Crème (O/W Emulsion)

| | | Gewichtsteile |
|---|---|---|
| A. | Panthenoltriester der p-Methoxyzimtsäure | 3 |
| | Vit. E-acetat | 2 |
| | Stearinsäure | 10 |
| | Cetylalkohol | 1 |
| | Glycerinmonomyristat | 5 |
| | Isopropylmyristat | 7 |
| | Oleylalkohol | 4 |
| B. | Diäthanolamin-cetylphosphat | 3 |
| C. | Wasser | 58 |
| | Panthenol | 1 |
| | Propylenglykol | 6 |
| | Parfüm, Konservierungsmittel | q.s. |

Die vereinigten Komponenten von A werden auf dem Wasserbad erhitzt, hierauf wird B bei dieser Temperatur zugegeben. Das Gemisch C wird auf 75° C erhitzt und zu A+B gegeben. Nach Abkühlung auf 25-30° C werden allfällige Wasserverluste kompensiert.

### D. Flüssiges Sonnenschutzöl

| | Gewichtsteile |
|---|---|
| Pantolactonester der p-Methoxyzimtsäure | 5 |
| Aethylalkohol | 20 |
| Oleylalkohol | 30 |
| Isopropylmyristat | 35 |
| Vit. E-acetat | 1 |
| Cetiol (Decyl-oleat) | 9 |
| | 100 |

### E. Sonnenschutzstift

| | Gewichtsteile |
|---|---|
| Pantolactonester der p-Methoxyzimtsäure | 1 |
| Panthenoltriester der p-Methoxyzimtsäure | 4 |
| CORHYDROL 1/35 (Hydriertes Rizinusöl) | 15 |
| Oleylalkohol | 20 |
| Texwax MH/81 (Mikrokristallines Wachs) | 30 |
| Mineralöl | 20 |
| Vaseline | 10 |

Die Komponenten werden bei 80° C unter Rühren geschmolzen und langsam abgekühlt. Bei 40° C wird die Masse in die gewünschten Formen gegossen.

**Patentansprüche**
**Patentsprüche für folgende Vertragsstaaten : BE, CH, LI, DE, FR, GB, IT, NL**

1. p-Methoxy-zimtsäureester der allgemeinen Formel

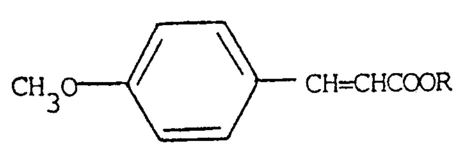

I

worin R den Alkoholrest von d,l-α-Tocopherol, Pantolacton oder Panthenol darstellt, wobei Panthenol ein-, zwei oder dreifach verestert sein kann.

2. d,l-α-Tocopherolester der p-Methoxy-zimtsäure.

3. Pantolactonester der p-Methoxy-zimtsäure.

4. Panthenoldiester der p-Methoxy-zimtsäure.

5. Panthenoltriester der p-Methoxy-zimtsäure.

6. Lichtschutzmittel, gekennzeichnet durch einen Gehalt an einem p-Methoxy-zimtsäureester der Formel

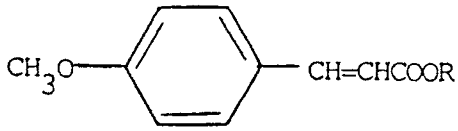

I

worin R den Alkoholrest von d,l-α-Tocopherol, Pantolacton oder Panthenol darstellt, wobei Panthenol ein-, zwei- oder dreifach verestert sein kann.

7. Verfahren zur Herstellung von p-Methoxy-zimtsäureestern der allgemeinen Formel

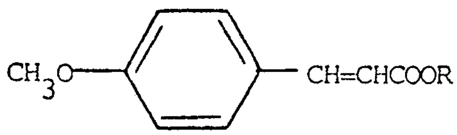

I

worin R den Alkoholrest von d,l-α-Tocopherol, Pantolacton oder Panthenol darstellt, wobei Panthenol ein-, zwei- oder dreifach verestert sein kann,
dadurch gekennzeichnet, dass man ein reaktives Derivat der p-Methoxyzimtsäure, insbesondere das Chlorid, in an sich bekannter Weise verestert.

8. p-Methoxy-zimtsäureestern der allgemeinen Formel

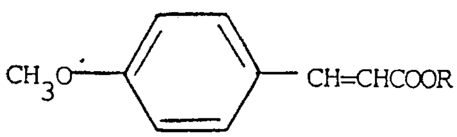

I

worin R den Alkoholrest von d,l-α-TocoPherol, Pantolacton oder Panthenol darstellt, wobei Panthenol ein-, zwei- oder dreifach verestert sein kann,
zur Verwendung als Lichtschutzmittel.

9. Verfahren zur Herstellung eines Lichtschutzmittels, dadurch gekennzeichnet, dass man p-Methoxy-zimtsäureester der allgemeinen Formel

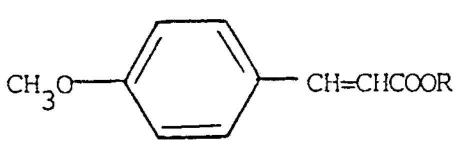

I

worin R den Alkoholrest von d,l-α-Tocopherol, Pantolacton oder Panthenol darstellt, wobei Panthenol ein-, zwei- oder dreifach verestert sein kann,
in eine in der Kosmetik übliche Grundlage einarbeitet.

**Patentansprüche für folgende Vertragsstaaten : ES, AT**

1. Lichtschutzmittel, gekennzeichnet durch einen Gehalt an einem p-Methoxy-zimtsäureester der Formel

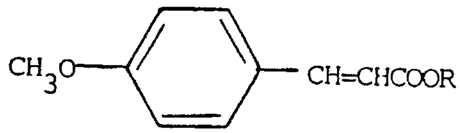

I

worin R den Alkoholrest von d,l-α-Tocopherol, Pantolacton oder Panthenol darstellt, wobei Panthenol ein-, zwei- oder dreifach verestert sein kann.

**2.** Lichtschutzmittel, gekennzeichnet durch einen Gehalt an dem d,l-α-Tocopherolester der p-Methoxy-zimtsäure.

**3.** Lichtschutzmittel, gekennzeichnet durch einen Gehalt an dem Pantolactonester der p-Methoxy-zimtsäure.

**4.** Lichtschutzmittel, gekennzeichnet durch einen Gehalt an dem Panthenoldiester der p-Methoxy-zimtsäure.

**5.** Lichtschutzmittel, gekennzeichnet durch einen Gehalt an dem Panthenoltriester der p-Methoxy-zimtsäure.

**6.** Verfahren zur Herstellung von p-Methoxy-zimtsäureestern der allgemeinen Formel

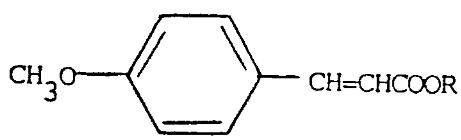

I

worin R den Alkoholrest von d,l-α-Tocopherol, Pantolacton oder Panthenol darstellt, wobei Panthenol ein-, zwei- oder dreifach verestert sein kann,
dadurch gekennzeichnet, dass man ein reaktives Derivat der p-Methoxyzimtsäure, insbesondere das Chlorid, in an sich bekannter Weise verestert.

**7.** p-Methoxy-zimtsäureestern der allgemeinen Formel

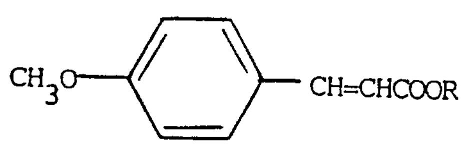

I

worin R den Alkoholrest von d,l-α-Tocopherol, Pantolacton oder Panthenol darstellt, wobei Panthenol ein-, zwei- oder dreifach verestert sein kann,
zur Verwendung als Lichtschutzmittel.

**8.** Verfahren zur Herstellung eines Lichtschutzmittels, dadurch gekennzeichnet dass man p-Methoxy-zimtsäureester der allgemeinen, Formel

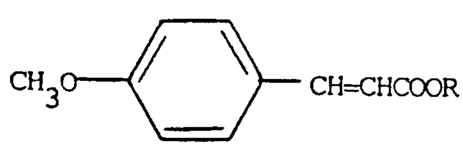

I

worin R den Alkoholrest von d,l-α-Tocopherol, Pantolacton oder Panthenol darstellt, wobei Panthenol ein-, zwei- oder dreifach verestert sein kann,
in eine in der Kosmetik übliche Grundlage einarbeitet.

## Claims

**Claims for the following Contracting States : BE, CH, LI, DE, FR, GB, IT, NL**

1. p-Methoxy-cinnamic acid esters of the general formula

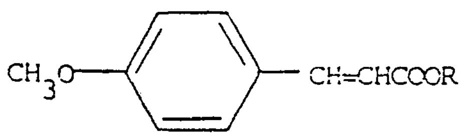

I

wherein R represents the alcohol residue of d,1-α-tocopherol, pantolactone or panthenol, whereby panthenol can be esterified once, twice or three times.

2. d,1-α-Tocopherol ester of p-methoxy-cinnamic acid.

3. Pantolactone ester of p-methoxy-cinnamic acid.

4. Panthenol diester of p-methoxy-cinnamic acid.

5. Panthenol triester of p-methoxy-cinnamic acid.

6. A light screening composition, characterized in that it contains a p-methoxy-cinnamic acid ester of the formula

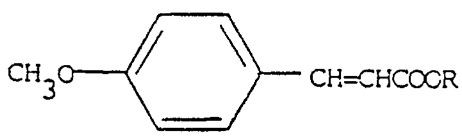

I

wherein R represents the alcohol residue of d,1-α-tocopherol, pantolactone or panthenol, whereby panthenol can be esterified once, twice or three times.

7. A process for the manufacture of p-methoxy-cinnamic acid esters of the general formula

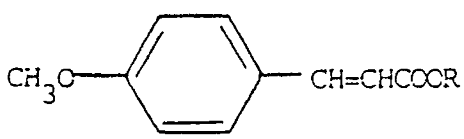

I

wherein R represents the alcohol residue of d,1-α-tocopherol, pantolactone or panthenol, whereby panthenol can be esterified once, twice or three times,
characterized by esterifying a reactive derivative of p-methoxycinnamic acid, especially the chloride, in a manner known per se.

8. p-Methoxy-cinnamic acid esters of the general formula

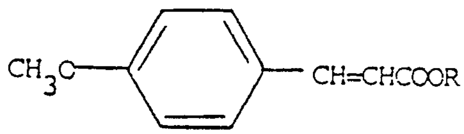

I

wherein R represents the alcohol residue of d,1-α-tocopherol, pantolactone or panthenol, whereby panthenol can be esterified once, twice or three times,
for use as light screening agents.

9. A process for the manufacture of a light screening composition, characterized by incorporating a p-methoxy-cinnamic acid ester of the general formula

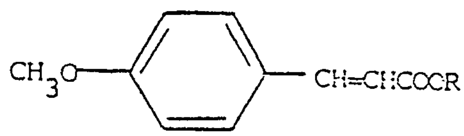

I

wherein R represents the alcohol residue of d,1-α-tocopherol, pantolactone or panthenol, whereby panthenol can be esterified once, twice or three times,
into a base which is usual in cosmetics.

**Claims for the following Contracting States : ES, AT**

1. A light screening composition, characterized in that it contains a p-methoxy-cinnamic acid ester of the general formula

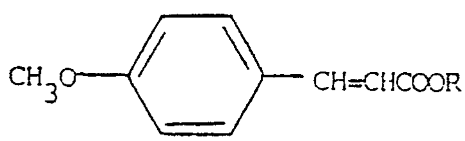

I

wherein R represents the alcohol residue of d,1-α-tocopherol, pantolactone or panthenol, whereby panthenol can be esterified once, twice or three times.

2. A light screening composition, characterized in that it contains the d,1-α-tocopherol ester of p-methoxy-cinnamic acid.

3. A light screening composition, characterized in that it contains the pantolactone ester of p-methoxy-cinnamic acid.

4. A light screening composition characterized in that it contains the panthenol diester of p-methoxy-cinnamic acid.

5. A light screening composition, characterized in that it contains the panthenol triester of p-methoxy-cinnamic acid.

6. A process for the manufacture of p-methoxy-cinnamic acid esters of the general formula

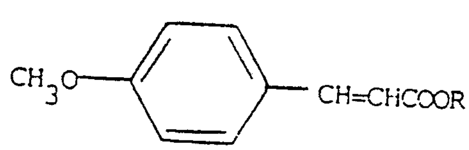

I

wherein R represents the alcohol residue of d,1-α-tocopherol, pantolactone or panthenol, whereby panthenol can be esterified once, twice or three times,
characterized by esterifying a reactive derivative of p-methoxycinnamic acid, especially the chloride, in a manner known per se.

**7.** p-Methoxy-cinnamic acid esters of the general formula

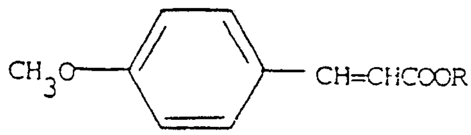

I

wherein R represents the alcohol residue of d,1-α-tocopherol, pantolactone or panthenol, whereby panthenol can be esterified once, twice or three times,
for use as light screening agents.

**8.** A process for the manufacture of a light screening composition, characterized by incorporating a p-methoxy-cinnamic acid ester of the general formula

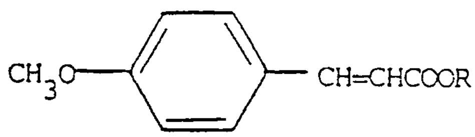

I

wherein R represents the alcohol residue of d,1-α-tocopherol, pantolactone or panthenol, whereby panthenol can be esterified once, twice or three times,
into a base which is usual in cosmetics.

**Revendications**
**Revendications pour les Etats contractants suivants : BE, CH, LI, DE, FR, GB, IT, NL**

**1.** p-méthoxy-cinnamate de formule générale

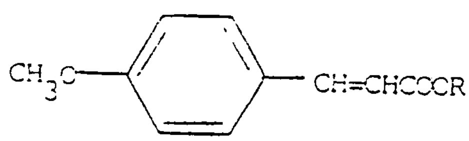

I

dans laquelle R représente le reste alcool de d,1-α-tocophérol, pantolactone ou panthénol, le panthénol pouvant être estérifié une, deux ou trois fois.

**2.** p-méthoxycinnamate de d,1-α-tocophérol.

**3.** p-méthoxycinnamate de pantolactone.

**4.** di-p-méthoxycinnamate de panthénol.

**5.** tri-p-méthoxycinnamate de panthénol.

**6.** Agent protecteur contre la lumière,
caractérisé par une teneur en p-méthoxycinnamate de formule

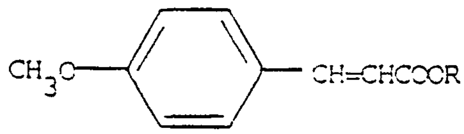

I

dans laquelle R représente le reste alcool de d,1-α-tocophérol, pantolactone ou panthénol, le panthénol pouvant être estérifié une, deux ou trois fois.

**7.** Procédé de préparation de p-méthoxycinnamates de formule générale

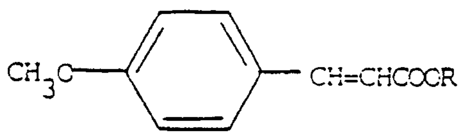

I

dans laquelle R représente le reste alcool de d,1-α-tocophérol, pantolactone ou panthénol, le panthénol pouvant être estérifié une, deux ou trois fois,
caractérisé en ce qu'on estérifié un dérivé réactif de l'acide p-méthoxycinnamique, notamment le chlorure, de la manière connue en soi.

**8.** p-méthoxy-cinnamates de formule générale

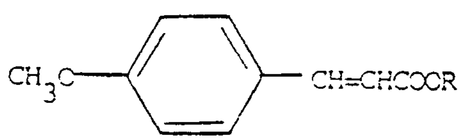

I

dans laquelle R représente le reste alcool de d,1-α-tocophérol, pantolactone ou panthénol, le panthénol pouvant être estérifié une, deux ou trois fois,
pour l'utilisation comme agent protecteur contre la lumière.

**9.** Procédé de préparation d'un agent protecteur contre la lumière, caractérisé en ce qu'on incorpore dans une base habituelle en cosmétique un p-méthoxycinnamate de formule générale

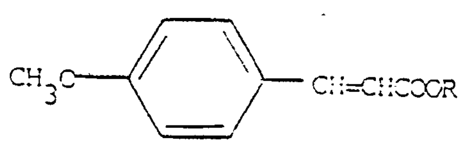

I

dans laquelle R représente le reste alcool de d,1-α-tocophérol, pantolactone ou panthénol, le panthénol pouvant être estérifié une, deux ou trois fois.

**Revendications pour les Etats contractants suivants : ES, AT**

**1.** Agent protecteur contre la lumière, caractérisé par une teneur en p-méthoxy-cinnamate de formule générale

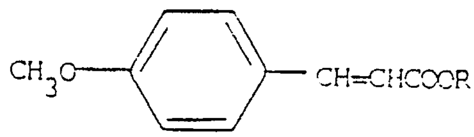 I

dans laquelle R représente le reste alcool de d,1-α-tocophérol, pantolactone ou panthénol, le panthénol pouvant être estérifié une, deux ou trois fois.

**2.** Agent protecteur contre la lumière, caractérisé par une teneur en p-méthoxycinnamate de d,1-α-tocophérol.

**3.** Agent protecteur contre la lumière, caractérisé par une teneur en p-méthoxycinnamate de pantolactone.

**4.** Agent protecteur contre la lumière, caractérisé par une teneur en di-p-méthoxycinnamate de panthénol.

**5.** Agent protecteur contre la lumière, caractérisé par une teneur en tri-p-méthoxycinnamate de panthénol.

**6.** Procédé de préparation de p-méthoxycinnamates de formule générale

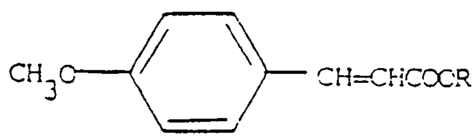 I

dans laquelle R représente le reste alcool de d,1-α-tocophérol, pantolactone ou panthénol, le panthénol pouvant être estérifié une, deux ou trois fois,
caractérisé en ce qu'on estérifie un dérivé réactif de l'acide p-méthoxycinnamique, notamment le chlorure, de la manière connue en soi.

**7.** p-méthoxy-cinnamates de formule générale

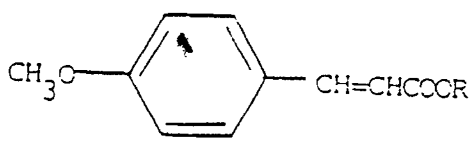 I

dans laquelle R représente le reste alcool de d,1-α-tocophérol, pantolactone ou panthénol, le panthénol pouvant être estérifié une, deux ou trois fois,
pour l'utilisation comme agent protecteur contre la lumière.

**8.** Procédé de préparation d'un agent protecteur contre la lumière, caractérisé en ce qu'on incorpore dans une base habituelle en cosmétique un p-méthoxycinnamate de formule générale

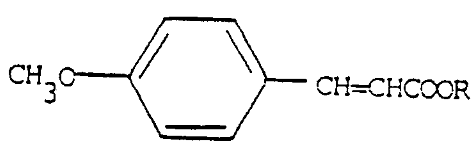 I

dans laquelle R représente le reste alcool de d,1-α-tocophérol, pantolactone ou panthénol, le panthénol pouvant être estérifié une, deux ou trois fois.